# EUROPEAN PATENT SPECIFICATION

(11) **EP 4 534 136 B1**
(45) Date of publication and mention of the grant of the patent: **22.07.2026**
(21) Application number: 23201552.9
(22) Date of filing: 04.10.2023
(51) Int. Cl.: A61N 1/32, A61N 1/362, A61N 1/365, A61N 1/05

(54) **AN APPARATUS, A METHOD, AND A COMPUTER PROGRAM PRODUCT FOR CONTROLLING CONDUCTION OF AN ELECTRICAL SIGNAL IN A HEART**
VORRICHTUNG, VERFAHREN UND COMPUTERPROGRAMMPRODUKT ZUR STEUERUNG DER LEITUNG EINES ELEKTRISCHEN SIGNALS IN EINEM HERZEN
APPAREIL, PROCÉDÉ ET PRODUIT DE PROGRAMME INFORMATIQUE POUR COMMANDER LA CONDUCTION D'UN SIGNAL ÉLECTRIQUE DANS UN COEUR

(43) Date of publication of application: 09.04.2025
(73) Proprietor: Stichting IMEC Nederland, 5656 AE Eindhoven (NL)
(72) Inventor: FICHMAN, Mark, 5654 KC Eindhoven (NL); MIHAJLOVIC, Vojkan, 5643 AZ Eindhoven (NL); HERMELING, Evelien, 6027 NT Soerendonk (NL)
(74) Representative: AWA Sweden AB

(56) References cited:
- US-A1- 2019 262 616
- US-A1- 2019 366 088
- US-B1- 10 485 977

## Description

### Technical field

The present description relates to controlling conduction of an electrical signal in a heart of a living being. The present invention provides an apparatus, and a computer program product for controlling conduction of the electrical signal in the heart, according to claims 1 and 13 respectively.

### Background

Beating of a heart provides a mechanical force for pumping of oxygenated blood to, and deoxygenated blood away from, peripheral tissue of a body. The beating of the heart depends critically on orderly activation and recovery of electrical excitation through myocardium of the heart. When the heart beats, electrical signals that cause the heart to contract, should follow a specific pathway through the heart. Any disruption in the signaling pathway can trigger an irregular heartbeat (arrhythmia). Arrhythmias can, in certain situations, increase the risk of a sudden cardiac arrest, which is an immediate life-threatening situation.

Therefore, in order to control arrhythmias, variety of treatments and tools have been developed, such as special medications and cardiac ablation. Cardiac ablation may be used for patients with severe conditions and uses heat or cold energy to create tiny scars in the heart to block irregular electrical signals and restore the typical heartbeat. Cardiac ablation is most often done by using thin and flexible tubes called catheters which are inserted through the veins or arteries.

Since ablation is, by definition, creating scars in the heart, it is also associated with risks, such as bleeding or infection at a site where the catheter was inserted, blood vessel damage, heart valve damage, new or worsening arrhythmia, slower heart rate, blood cloths, stroke or heart attack, veins narrowing, kidney damage from injection of contrast material during the procedure, and in some rare situations also death.

Cardiac ablation creates permanent scars, which imply that a permanent change to electrical pathways through the heart is created. The blocked pathways cannot be restored. Hence, cardiac ablation needs to be carefully planned by a cardiac electrophysiologist and needs to be customized for each patient.

There is a need for an improved control of conduction of electrical signals in the heart. In particular, such improved control would be very useful for patients that would presently need to undergo a cardiac ablation procedure.

An apparatus according to the preamble of claim 1 is disclosed in US 10 485 977 B1.

### Summary

An objective of the present description is to provide control of conduction of electrical signals in the heart. In particular, an objective of the present description is to provide a control of conduction that may be dynamically changed and that is reversible.

These and other objectives of the present description are at least partly achieved by the invention as defined in the independent claims. Preferred embodiments are set out in the dependent claims.

According to a first aspect, there is provided an apparatus for controlling conduction of an electrical signal in a heart, said apparatus comprising: a stimulation generating unit configured to generate a first stimulation signal and a second stimulation signal, wherein the first stimulation signal has a first frequency and the second stimulation signal has a second frequency; a first pair of electrodes configured to be arranged in relation to a location in the heart in which conduction of the electrical signal is to be controlled, wherein the first pair of electrodes is configured to receive the first stimulation signal; a second pair of electrodes configured to be arranged in relation to the location in the heart in which conduction of the electrical signal is to be controlled, wherein the second pair of electrodes is configured to receive the second stimulation signal, wherein the first and second pairs of electrodes are configured to form an interferential stimulation signal in the location of the heart based on the first and second stimulation signals, wherein a difference between the first frequency and the second frequency defines a beat frequency of the interferential stimulation signal for controlling conduction of the electrical signal in the location in the heart; an electrical signal sensor configured to detect conduction of the electrical signal in the heart; and a control unit configured to receive input from the electrical signal sensor and configured to control output of the first and the second stimulation signals for controlling a timing of the interferential stimulation signal in relation to the conduction of the electrical signal in the heart.

Thus, thanks to the first aspect, an apparatus is provided which may output a stimulation signal that affects conduction of electrical signals in the heart. The stimulation signal may be controlled such that the stimulation signal is only output when needed. The stimulation signal may interact with electrical signals being conducted in the heart so as to affect such electrical signals. However, the stimulation signal may be configured not to affect tissue such that no scars or any permanent damage to tissue are created. This implies that the apparatus may directly affect the condition that needs to be treated (undesired conduction of electrical signals) without a need to physically prevent any conduction of electrical signals.

Thus, the apparatus is less harmful to a patient compared to use of cardiac ablation. Since no scars are formed in tissue, there is substantially lower risks of blood vessel damage, heart valve damage, blood cloths, stroke or heart attack being caused by implantation of the apparatus. In addition, scars may affect movement of the heart muscle so as to impair the ability of the heart to contract and expand, whereas the apparatus of the first aspect does not affect movement of the heart muscle.

The conduction of electrical signals in the heart is controlled by an interferential stimulation signal provided by the apparatus. Thus, the apparatus is configured to generate a first and a second stimulation signal, which will interfere in the location in the heart to form the interferential stimulation signal that affects the conduction of the electrical signals. This implies that the control of conduction of electrical signals is not tied to the exact placement of electrodes. For instance, since the stimulation signal output by an electrode does not directly affect the conduction of electrical signals, the apparatus may mainly affect tissue that is spaced apart from the electrodes. This implies that a very accurate control of a portion within the location in which the interferential stimulation signal affects electrical signals within the heart may be provided. Thus, the apparatus is configured such that a strongest effect on electrical signals is not provided closest to the electrodes. Rather, the apparatus may provide an interferential stimulation signal that only affects a target portion spaced apart from the electrodes, without affecting tissue between the electrode and the target portion.

The first and the second stimulation signals may be configured so as not to individually affect conduction of electrical signals in the heart. For instance, intensity of the individual first and second stimulation signals may be low. Further, the first and second frequencies of the first and second stimulation signals, respectively, may be too high in order to affect the electrical signals that are conducted in the heart.

However, the first and the second frequencies are different. Thus, the first and second stimulation signals generated by the stimulation generating unit enables an interferential stimulation signal to be formed, wherein interference of the first and second stimulation signals defines a beat frequency based on a difference between the first frequency and the second frequency. The beat frequency may hence be much lower than the first frequency and the second frequency, respectively, and may be adapted to affect the conduction of electrical signals in the location in the heart.

The beat frequency may be defined such that the interferential stimulation signal may exhibit a waveform that affects conduction of the electrical signal in the heart. Thus, the stimulation generating unit may be adapted to output the first stimulation signal and the second stimulation signal such that electrical signals in the heart are affected by the interference formed between the first and second stimulation signals.

The first frequency and the second frequency may be selected to be high frequencies, whereas the beat frequency represents a low frequency lower than the first frequency and the second frequency. The heart may be affected to a larger extent by low frequency signals than high frequency signals. Thus, the first stimulation signal and the second stimulation signal may include a first frequency and a second frequency, respectively, which do not affect, or minimally affect, the heart. Further, an amplitude of the first stimulation signal and an amplitude of the second stimulation signal may be smaller than an amplitude of an interferential signal with the beat frequency, such that a sufficiently large signal (amplitude) for affecting conduction of electrical signals in the heart is only provided by the interferential signal.

According to an embodiment, the stimulation generating unit is configured to generate a first frequency and a second frequency, respectively, being in a range of 500 Hz - 1 MHz, such as 5 kHz - 100 kHz.

Thus, the stimulation generating unit may generate signals of the first stimulation signal and the second stimulation signal having a relatively high frequency. The first frequency and the second frequency may be so high as not to affect, or minimally affect, the heart. By selecting these frequencies to be high, such as in the range of 5 kHz - 1 MHz or in the range of 5 kHz - 100 kHz, the heart is not stimulated by the first stimulation signal and the second stimulation signal individually. Rather, stimulation of the heart may be provided solely by the interferential stimulation signal between the first stimulation signal and the second stimulation signal.

The first frequency and the second frequency may, while not causing generation of signals heart, be configured to block a signal transported by the heart. In this case, the first frequency and the second frequency may for instance be in a range of 5 kHz - 100 kHz

The first frequency and the second frequency may preferably not be too high so that a relatively small difference in the frequencies may still be accurately defined. Thus, the first frequency and the second frequency may be lower than 1 MHz, such as lower than 100 kHz.

According to an embodiment, the stimulation generating unit is configured to generate the first frequency and the second frequency with a difference between the first frequency and the second frequency being in a range of 1 Hz - 10 kHz, such as 1 - 5 kHz.

Thus, the beat frequency may be in the range of 1 Hz - 10 kHz or in the range of 1 - 5 kHz. The use of a beat frequency in these ranges may be suitable for providing control of conduction of electrical signals in the heart. If the beat frequency is very low, it may be difficult to accurately define the interferential signal in relation to high frequencies of the first and the second frequencies. Thus, the beat frequency may preferably be higher than 1 Hz or higher than 1 kHz. If the beat frequency is too high, the interferential signal may not provide a strong effect for controlling conduction of electrical signals in the heart. Thus, the beat frequency may preferably be lower than 10 kHz or lower than 5 kHz. The beat frequency may be selected in relation to a desired stimulation of the heart.

The conduction of electrical signals in the heart may be provided as part of the cardiac conduction system controlling the function of the heart muscle. The electrical signals may thus be based on an electrical impulse generated in the sinoatrial node, which impulse is then propagated through the heart. The electrical impulse in a muscle cell of the heart may form an action potential that may rise, be high for a short duration, and then fall again. This action potential may further be passed to adjacent muscle cells so as to propagate the electrical signal through the heart.

As used herein, conduction of electrical signals in the heart should be construed as including the propagation of action potentials within the heart that control the function of the heart to cause expansion and contraction of the heart. However, conduction of electrical signals in the heart may refer to any action potentials occurring in muscle cells, which may or may not relate to normal heart function.

It should be realized that the apparatus may be configured to affect the location in the heart so as to trigger an electrical signal to be generated in the location. This may be used for promoting electrical activity in desired locations, which may reinforce desired electrical signals along desired pathways through the heart over electrical signals following undesired pathways through the heart. However, the apparatus need not necessarily trigger generation of electrical signals but may rather be used for blocking electrical signals so as to stop propagation of electrical signals along undesired pathways. Thus, the term *"stimulation signal"* mean that a signal is provided that stimulates the heart to cause a desired effect in the location in the heart, which may imply triggering generation or propagation of an electrical signal in the heart or preventing or blocking of an electrical signal in the heart.

As used herein, the term *"location"* in which stimulation is to be provided should be construed as a region within the heart that is addressed by the stimulation signals. The first and second pairs of electrodes may be arranged close to the location. It should be realized that the location may not have clear boundaries but is rather a region within heart tissue in which stimulation signals may propagate and in which an interferential stimulation signal is formed. It should further be realized that the stimulation signals may be controlled so as to steer an exact portion of the location in which the interferential stimulation signal provides stimulation of the heart.

The apparatus of the first aspect may be configured to provide the interferential stimulation signal for causing an interaction with electrical activity in muscle cell(s) of the heart. The apparatus may be controlled such that the interferential stimulation signal may be provided at a desired time, such that the interferential stimulation signal may provide a desired effect. Thus, the timing of the interferential stimulation signal may for instance ensure that the interferential stimulation signal is provided when an undesired electrical signal passes the location in the heart, such that the interferential stimulation signal interacts with the undesired electrical signal and blocks the undesired electrical signal from being further conducted through the heart.

The timing of the interferential stimulation signal may alternatively ensure that the interferential stimulation signal is provided when a desired electrical signal is to pass the location in the heart, such that the interferential stimulation signal may trigger a desired electrical signal to be generated in the location and be further conducted through the heart.

The apparatus comprises the electrical signal sensor that detects electrical signals in the heart. The input from the electrical signal sensor may be used by the control unit to ensure that the interferential stimulation signal is properly timed. Thus, the apparatus is able to identify a timing of the interferential stimulation signal so as to adapt the interferential stimulation signal to the electrical signals being conducted through the heart.

The electrical signal sensor may be configured to be arranged in relation to the location in the heart, such as close to the first and second pairs of electrodes. Thus, the electrical signal sensor may be configured to detect electrical signals close to the location in the heart to be stimulated, which may for instance be used for timing the interferential stimulation signal so as to block conduction of the electrical signal. The electrical signal sensor may alternatively be configured to detect electrical signals in an area of the heart which is not necessarily in immediate vicinity to the location of the heart. The control unit may be able to determine when a signal is to be generated or blocked in the location affected by the interferential stimulation signal, such that the control unit may provide the interferential stimulation signal with a desired timing.

The control unit may further determine whether any control of conduction of electrical signals is needed or not. For instance, the apparatus may only need to provide stimulation when an abnormal condition, such as arrhythmia is ongoing. A patient having the apparatus implanted need not necessarily have continuous arrhythmia problems. Rather, it may be an intermittent condition. Thus, the apparatus may be configured to continuously detect electrical signals in the heart but may only activate stimulation when needed.

It should be realized that the stimulation generating unit may be controlled such that the interferential stimulation signal may be steered. Thus, by controlling the first and second stimulation signals, the location in which the interferential stimulation signal may affect electrical signals in the heart may be moved.

It should further be realized that the interferential stimulation signal may be further controlled by using further electrodes. For instance, the stimulation generating unit may be controlled so as to control to which electrodes the first and second stimulation signals are output. The apparatus may comprise at least three electrodes such that the first pair of electrodes may be selected and dynamically defined from these at least three electrodes, so as to change a position at which the first stimulation signal is output. Similarly, the second pair of electrodes may be selected and dynamically defined from at least three other electrodes, so as to change a position at which the second stimulation signal is output. Also, the apparatus may comprise an array of electrodes allowing the first and second pair of electrodes to be selected and dynamically defined within the array of electrodes. By controlling a position at which the first and/or second stimulation signals are output, the portion of the location in which stimulation by the interferential stimulation signal is provided may be steered.

Further, the apparatus may comprise a third pair of electrodes, which may be formed by a dedicated pair of electrodes or may be selected and dynamically defined within an array of electrodes as discussed above for the first and second pair of electrodes. The stimulation generating unit may further be configured to generate a third stimulation signal having a third frequency, which may be the same as the first frequency or the second frequency or may be different from both the first frequency and the second frequency.

The third pair of electrodes may be configured to be arranged in relation to the location in the heart in which conduction of the electrical signal is to be controlled, wherein the third pair of electrodes is configured to receive the third stimulation signal. The third stimulation signal may further contribute to forming the interferential stimulation signal in the location of the heart, such that the interferential stimulation signal is based on the first, second, and third stimulation signals. The third stimulation signal may be used for further controlling the interferential stimulation signal formed by the first and second stimulation signals.

In an embodiment, the third stimulation signal may have a frequency corresponding to a multiple of the beat frequency. The third stimulation signal may be used for controlling a portion of the location in which stimulation is provided for controlling conduction of the electrical signal in the location in the heart.

The third stimulation signal may be configured to modulate a beat frequency signal defined by interference of the first stimulation signal and the second stimulation signal such that an amplitude of the beat frequency signal is substantially increased. In particular, the third frequency may be related to the beat frequency by an integer number. Thus, the third stimulation signal may be matched to the beat frequency signal in order to amplify the beat frequency signal and to control conduction of electrical signals in the heart. A phase of the third stimulation signal may be controlled in relation to a phase of the beat frequency signal such that constructive interference may be achieved so as to cause interferential stimulation in the location of the heart.

According to an embodiment, the beat frequency is two times larger than the third frequency.

This facilitates modulating the interference between the first and second stimulation signals so as to form an interferential signal with a similar signal shape as a typical monophasic or biphasic current stimulation which is realized in existing implants. Thus, a signal may be formed having two parts with opposite signs.

According to an alternative embodiment, the third frequency may be equal to the beat frequency, or the third frequency may be two, three or four times larger than the beat frequency. This may still allow the third stimulation signal to modulate the interference between the first and second stimulation signals so as to form an interferential stimulation signal that may efficiently cause stimulation in the heart.

It should be realized that the apparatus may further comprise additional pairs of electrodes arranged in relation to the location in the heart, wherein the stimulation generating unit is configured to generate further stimulation signals to the additional pairs of electrodes for controlling the interferential stimulation signal using even further stimulation signals.

The apparatus may be used for controlling conduction of electrical signal in a heart of any living being. It should be realized that the apparatus may be used for controlling conduction of electrical signals in a heart of a human being, which may be referred to herein as a subject or a patient. Although the apparatus is mainly discussed herein in relation to being used for controlling conduction of electrical signals in a heart of a human being, it should be understood that the apparatus may also or alternatively be used for controlling conduction of electrical signal in a heart of an animal.

According to the invention, the apparatus is configured to be implanted in a body of the subject. According to further comparative examples, the apparatus is configured to be at least partially implanted in the living being with the first pair of electrodes and the second pair of electrodes arranged differently in relation to the location in the heart.

Thus, the apparatus is adapted to provide the pairs of electrodes close to or in contact with the location in the heart to be stimulated. This implies that stimulation signals may be output by the apparatus directly into the heart providing an accurate control of the stimulation within the heart.

The apparatus may be configured to be completely implanted within the body of the living being. This facilitates use of the implanted apparatus for providing stimulation of the heart to the living being in a convenient way. In particular, when the apparatus is implanted within the body of a human being, the stimulation of the heart may be provided with minimal effect of everyday life of the human being for providing the stimulation.

The apparatus may be configured to be partially implanted within the body of the living being. Thus, the first pair of electrodes and the second pair of electrodes may be implanted within the body. The pairs of electrodes may further be configured to receive stimulation signals from the stimulation generating unit being implanted. Thus, the stimulation generating unit may also be implanted. This implies that there may be no need for wires connecting the stimulation generating unit to pairs of electrodes through the skin of the living being.

Parts of apparatus being implanted may be configured to communicate with parts of the apparatus being arranged externally to the living being. If the apparatus is completely implanted, the apparatus may still be configured to communicate with an external unit. Communication between implanted parts and parts arranged externally to the living being may be achieved through wireless communication.

For instance, a fully implanted apparatus may receive control signals from the external unit for changing parameters of the stimulation, such as parameters defining a stimulation paradigm, such as to fine-tune the portion of the location within the heart to be stimulated or a frequency, an amplitude, or a duration of a stimulation to be provided.

The externally arranged device may be provided in a housing that may be worn by the living being or may be adapted for being arranged in vicinity of the living being. The implanted apparatus or implanted parts of the apparatus may be configured to communicate with a communication unit, which may be provided in a housing worn by the living being or arranged in vicinity of the living being, wherein the communication unit may act as an intermediate device for allowing the implanted apparatus to communicate with an external unit which may be remotely arranged. Thus, communication between the communication unit and the external unit may be provided via a telecommunication or computer network.

The control unit may be arranged in a separate housing from the stimulation generating unit. The control unit may then for instance be arranged in a housing that may be configured to be worn by the living being. The control unit may then be configured to provide control of the stimulation generating unit being implanted through wireless communication. However, the control unit may preferably be arranged in a common housing with the stimulation generating unit in order to ensure accurate control of the first stimulation signal and the second stimulation signal.

Thus, the apparatus may comprise a plurality of physical units which may or may not be connected by wires. If parts of the apparatus are not physically connected, wireless communication may be used to allow the parts to communicate. For instance, the control unit may be arranged in one housing and may communicate control signals wirelessly to the stimulation generating unit being arranged in another housing.

According to an embodiment, the control unit is configured to control output of the first and the second stimulation signals for controlling the interferential stimulation signal to block conduction of the electrical signal in the heart.

This is useful for ensuring that electrical signals are not conducted along undesired pathways in the heart. This may be very helpful in preventing arrhythmia in the subject.

It should be realized that the interferential stimulation signal may not necessarily completely block conduction of the electrical signal but may instead attenuate the electrical signal.

According to an embodiment, the control unit is configured to control output of the first and the second stimulation signals for controlling the interferential stimulation signal to trigger conduction of the electrical signal in the heart.

This is useful for ensuring that electrical signals are triggered and generated in desired pathways in the heart. The triggering of electrical signals may be used for strengthening electrical signals in the desired pathways such that a desired behavior of the heart may be reinforced.

The apparatus may advantageously be used for both blocking or attenuating conduction of electrical signals in undesired pathways and triggering and/or strengthening conduction of electrical signals in desired pathways. This may ensure that a proper conduction of electrical signals may be provided in the heart.

According to an embodiment, the stimulation generating unit is configured to generate a pulse of the first stimulation signal and a pulse of the second stimulation signal for forming an interferential stimulation signal having a duration of not more than two periods of the beat frequency, such as a single period or half a period of the beat frequency.

Thus, the stimulation generating unit may be configured to generate pulses of the first and second stimulation signals with short duration. In particular, the stimulation generating unit may be configured to generate pulses of the first and second stimulation signals corresponding to half a period of the beat frequency. This implies that the interferential stimulation signal may be provided having a rise in intensity or a fall in intensity, which may be sufficient to provide desired stimulation.

The desired effect may be provided also if the interferential stimulation signal is provided for a duration longer than half a period of the beat frequency. For instance, the interferential stimulation signal may be provided for a full period of the beat frequency, including a rise in intensity followed by a fall in intensity. This may correspond to the action potential of an electrical signal in the heart. This may therefore be suited for stimulating, such as blocking or triggering, the electrical signal in the heart.

It may be beneficial to provide a short pulse of the first and second stimulation signals to ensure a low power consumption of the apparatus. Also, it may be beneficial to provide a short pulse of the first and second stimulation signals so as to ensure that stimulation of the heart is only applied when needed and, for instance, to ensure that undesired triggering of electrical signals in the heart is not provided.

It should be understood that the term *"pulse"* is used herein to refer to a short duration of the stimulation signals and is hence a characteristic of the stimulation signal. Unless explicitly stated, it does not refer to a characteristic of the heart (such as heart rate).

It should be realized that the apparatus may be configured to individually detect a need for stimulation during each beat of the heart. However, in case of arrhythmia, there may be an ongoing condition for a period of time. This implies that undesired electrical signals may be repetitively occurring but with an unpredictable frequency or rate. Thus, the apparatus may be configured to detect a sequence of undesired electrical signals by detecting each undesired electrical signal in the sequence individually and providing the first and second stimulation signals to cause interferential stimulation for blocking each electrical signal individually.

The apparatus may be configured to provide stimulation to trigger generation of electrical signals in the heart based on a desired heart rate. Thus, the stimulation to trigger electrical signals may be based on detecting a heart rate, wherein pulses of the first and second stimulation signals may not need to be individually controlled but may rather be set by defining a frequency of the pulses of the stimulation signals. However, it should be realized that for stimulation to trigger generation of electrical signals in the heart, each pulse of the first and second stimulation signal may be individually controlled.

According to an embodiment, the control unit is configured to store a model for determining the timing of the interferential stimulation signal in relation to the input from the electrical signal sensor or the control unit is configured to trigger output of the first and second stimulation signals based on detection of conduction of the electrical signal in the heart.

The electrical signal sensor may be arranged to monitor electrical signals in a detection location close to the location that is stimulated by the interferential stimulation signal. The control unit may further be configured to trigger output of the first and second stimulation signals with a fixed delay in relation to the electrical signal being detected by the electrical signal sensor. The fixed delay may be determined based on calibration of the apparatus when the apparatus is implanted in the subject. This is a simple manner of controlling the stimulation and ensuring that the apparatus provides a stimulation that is properly timed to the conduction of electrical signals in the heart. For instance, the apparatus may be configured to form the interferential stimulation signal such that the interferential stimulation signal will block the conduction of the electrical signal in the heart at the location being stimulated.

The control unit may also or alternatively use a more complex relation between the timing of the interferential stimulation signal and the detected electrical signals. Such complex relation may be represented by the stored model. For instance, the apparatus may be configured to detect electrical signals in one or more detection locations in the heart. The control unit may use an algorithm that determines the timing of the interferential stimulation signal using the stored model. For instance, the model may represent a temporal profile of activation of a single location or multiple locations in the heart, such that the algorithm may determine a timing for triggering the interferential stimulation based on detected electrical signals. The temporal profile may for instance be based on previously detected activations of the location(s) in the heart. The model may be used for controlling stimulation in a closed loop manner such that timing of stimulation may be adjusted between different heart beats for achieving a desired behavior of the heart (e.g., normal heart rhythm without arrhythmias).

According to an embodiment, the electrical signal sensor comprises multiple sensor units configured to detect electrical signals in multiple locations in the heart.

This implies that the apparatus is able to detect electrical signals so as to determine a pattern of conduction of electrical signal in the heart. Thus, the control unit may determine the timing of the interferential stimulation signal to ensure that the timing of the interferential stimulation signal fits an activation pattern as detected by the multiple sensor units.

According to an embodiment, the electrical signal sensor comprises electrodes configured to be arranged at the location of the heart for detecting electrical signals conducted in the location of the heart.

Thus, the electrical signal sensor may be configured to detect electrical signals in the location that is also stimulated by the stimulation signals output by the first and second pairs of electrodes. This may be useful in enabling the control unit to trigger output of the first and second stimulation signals based on detection of an electrical signal in the location that is to be stimulated.

According to an embodiment, the stimulation generating unit comprises a first signal generator associated with the first pair of electrodes for generating the first stimulation signal and outputting the first stimulation signal to the first pair of electrodes and wherein the stimulation generating unit comprises a second signal generator associated with the second pair of electrodes for generating the second stimulation signal and outputting the second stimulation signal to the second pair of electrodes.

Thus, the stimulation generating unit may comprise a first and a second signal generator, each dedicated to generating the first stimulation signal and the second stimulation signal, respectively. This implies that the first stimulation signal to be output to the first pair of electrodes and the second stimulation signal to be output to the second pair of electrodes may be separately controlled to ensure that desired stimulation signals are output, and that the first stimulation signal is not affected by generation of the second stimulation signal in the stimulation generating unit, and vice versa. Hence, the interferential stimulation may be accurately controlled by separately controlling the first stimulation signal and the second stimulation signal.

According to an embodiment, the control unit is configured to control modulation of the interferential stimulation signal by modulating the first frequency and/or the second frequency, by modulating an amplitude of the first stimulation signal and/or an amplitude of the second stimulation signal, and/or by modulating a time instant for start of the first stimulation signal and/or a time instant for start of the second stimulation signal.

The control unit may be configured to control the stimulation generating unit for controlling the stimulation of the location in the heart. The control unit may be configured to transmit control signals to the stimulation generating unit for controlling the stimulation generating unit and modulating the stimulation signals generated by the stimulation generating unit.

The control unit may be configured to control timing of output of the stimulation signals to the first pair of electrodes and the second pair of electrodes. As mentioned above, the stimulation generating unit may comprise a plurality of signal generators, each dedicated to generating the first stimulation signal and the second stimulation signal, respectively. Thus, the control unit may be configured to provide synchronization of the signal generators to ensure that the first stimulation signal and the second stimulation signal are output with a desired time relation.

The first signal generator and the second signal generator may each be configured to generate an alternating signal. The control unit may be configured to provide a control signal to the first signal generator and the second signal generator, respectively, for modulating the alternating signal output by the first signal generator and the second signal generator, respectively.

The control unit may be configured to modulate the first frequency of the first stimulation signal and/or to modulate the second frequency of the second stimulation signal. This implies that the beat frequency may be controlled, which may be used for controlling stimulation of the location in the heart.

The control unit may also or alternatively be configured to modulate an amplitude of the first stimulation signal and/or modulate an amplitude of the second stimulation signal. This implies that the portion of the location of the heart having a maximum amplitude of the interferential signal may be fine-tuned. If the amplitude of the first stimulation signal is increased, the portion of the location having maximum amplitude of interference between the first stimulation signal and the second stimulation signal is moved closer to the second pair of electrodes, and vice versa. Thus, by modulating the amplitude of the first stimulation signal and/or the amplitude of the second stimulation signal, the portion of the location within the heart where stimulation is provided may be fine-tuned and dynamically changed. This may be particularly useful for allowing the stimulation to be calibrated when the apparatus is implanted in order to provide desired stimulation of the heart.

The control unit may also or alternatively be configured to modulate a time instant for start of the first stimulation signal and/or a time instant for start of the second stimulation signal, respectively. Thus, the control unit may be configured to control a phase relation between the stimulation signals, which further controls a waveform of the interferential signal.

It should further be realized that the interferential stimulation signal may be further controlled by using further electrodes. For instance, the stimulation generating unit may be controlled so as to control to which electrodes the first and second stimulation signals are output. The control unit may thus be configured to control modulation of the interferential stimulation signal by controlling which electrodes are used for output of the stimulation signals. In addition, the control unit may be configured to control modulation of the interferential stimulation signal by controlling whether to activate the stimulation generating unit to output a third (or further) stimulation signal to a third (or further) pair of electrodes at the location of the heart.

According to an embodiment, the apparatus further comprises a set of additional electrodes, wherein the set of additional electrodes comprises at least two pairs of electrodes configured to be arranged in relation to an additional location in the heart, wherein each pair of electrodes is configured to receive a respective stimulation signal for forming an interferential stimulation signal in the additional location of the heart based on the stimulation signals.

It should be realized that the set of additional electrodes is different from the electrodes described above. Thus, the first pair of electrodes and the second pair of electrodes previously described may form part of a first set of electrodes. The set of additional electrodes may then form a second set of electrodes different from the first set of electrodes. The first set of electrodes may be configured to be arranged in relation to a first location in the heart and the second set of electrodes may be configured to be arranged in relation to a second location in the heart, different from the first location.

Thus, the apparatus may be configured to provide stimulation in multiple locations of the heart, wherein a respective set of electrodes is dedicated to each location of the heart. The stimulation in each location of the heart may be provided based on respective stimulation signals. Thus, each set of electrodes may be associated with a respective stimulation generating unit or a single stimulation generating unit is at least able to generate separate stimulation signals to different sets of electrodes.

The control unit may be configured to control timing of the interferential stimulation signal in each of the locations of the heart being stimulated. The control unit may be configured to control the timing such that the interferential stimulation signals provided in the locations are properly timed in relation to each other. Thus, the control unit may ensure that the conduction of electrical signals in the heart follows a desired pattern through the heart by the apparatus providing stimulation in multiple locations with the stimulation in different locations being properly timed in relation to each other.

However, it should be realized that the control unit may be configured to individually control the timing of the interferential stimulation signal in each of the locations. For instance, the apparatus may be used for blocking or preventing conduction of local electrical signals in multiple locations and the blocking of electrical signals in different locations may be unrelated.

It should further be realized that, while the apparatus may need to use interferential stimulation for blocking conduction of the electrical signal in the heart, this may be combined with the apparatus being configured to output a single stimulation signal for triggering conduction of the electrical signal in the heart.

Thus, triggering conduction of the electrical signal may be controlled by a simple stimulation signal, which may not necessarily be controlled to a particular location based on interference between two stimulation signals. Thus, the apparatus could include, in addition to the first and second pair of electrodes, an additional pair of electrodes configured to be arranged in relation to an additional location in the heart, wherein the additional pair of electrodes is configured to receive a stimulation signal for triggering conduction of an electrical signal in the additional location of the heart based on the stimulation signal.

In particular, such stimulation signal to the additional pair of electrodes may be paced with respect to the cardiac cycle.

According to an embodiment, each pair of electrodes is arranged on a flexible, elongate carrier, wherein the carrier comprises wires for connecting each electrode to the stimulation generating unit.

The apparatus may be implanted in the subject. The apparatus may comprise a base unit, which may be arranged in a suitable position close to the heart. The base unit may comprise a housing, in which the stimulation generating unit and the control unit may be arranged.

The apparatus may further comprise carriers on which electrodes are arranged. The carriers may further be connected to the base unit in order to connect the pairs of electrodes to the stimulation generating unit in order to allow the stimulation signals to be output to the pairs of electrodes.

Thanks to using a flexible, elongate carrier, the apparatus may allow freely placing the electrodes in relation to desired locations in the heart and also freely arranging the base unit in the subject.

Each pair of electrodes may be arranged on a single carrier. This may allow the electrodes of a pair to be positioned using a single carrier, which may simplify placement of the electrodes during implantation of the apparatus. For instance, the electrodes of the pair of electrodes may be arranged at different positions along a longitudinal extension of the flexible carrier.

However, having the pair of electrodes on a single carrier provides a lower degree of freedom in placing the electrodes within a pair in relation to each other. Thus, in another embodiment, each electrode may be configured to be separately arranged in relation to a respective location in the heart.

According to an embodiment, the apparatus further comprises a sensor for detecting information relating to functionality of the heart and a processor configured to receive and analyze the information detected by the sensor and to provide input to the control unit for adapting control by the control unit.

Thus, the apparatus may be configured to control stimulation based on additional information relating to functionality of the heart. For instance, the apparatus may be configured to detect an electrocardiogram (ECG) for monitoring functionality of the heart. The ECG may be analyzed by the processor for identifying whether there is a need for stimulation to control the conduction of electrical signals in the heart.

The processor may be configured to identify the need for stimulation. The processor may also be configured to identify a need for adapting the stimulation, such as adapting a timing of interferential stimulation signal(s) or fine-tuning the portion within the location of the heart in which the interferential stimulation signal provides stimulation of the heart.

According to an embodiment, the apparatus is configured to be implanted in a body of a subject, such as the apparatus forming part of a cardiac resynchronization therapy (CRT) device or an implantable cardioverter defibrillator (ICD) device.

This implies that the apparatus may be arranged in close relation to the location in the heart to be stimulated. Thus, stimulation signals may be output by the apparatus directly into the heart providing an accurate control of the stimulation within the heart.

According to a second aspect of the disclosure, there is provided a method for controlling conduction of an electrical signal in a heart, said method comprising: receiving input from an electrical signal sensor configured to detect conduction of the electrical signal in the heart; determining a timing of an interferential stimulation signal in relation to the conduction of the electrical signal in the heart; and outputting one or more control signals for controlling a first stimulation signal and a second stimulation signal in dependence of the determined timing.

The method relates to controlling generation of signals that may be used for stimulation of the heart. The method provides control signals for controlling timing of generation of the first stimulation signal and the second simulation signal. Thus, the method provides control of an apparatus for generating stimulation signals. The method does not relate to actual output of the stimulation signals and is therefore not related to a method of treatment of a living being.

Thanks to receiving input of conduction of electrical signals in the heart, the method is able to determine control signals that may be used for ensuring that the timing of stimulation is adapted to the electrical signals being conducted in the heart.

According to a third aspect, there is provided a computer program product comprising computer-readable instructions such that when executed on a processing unit, the computer program product will cause the processing unit to perform the method according to the second aspect.

Effects and features of this third aspect are largely analogous to those described above in connection with the first and second aspects. Embodiments mentioned in relation to the third aspect are largely compatible with the first and second aspects.

The computer program product may implement the method in a processing unit, which may be a dedicated processing unit for performing the method or may be a general-purpose processing unit which may be able to perform the method based on the computer program product. For instance, the processing unit may be embedded in a small, implantable apparatus.

The computer program product may comprise a computer-readable medium on which the computer-readable instructions are stored. The computer program product may thus be provided as a non-transient computer program product stored on any tangible medium. Alternatively, the computer program product may be provided as a signal carrying the computer-readable instructions for allowing the computer program product to be loaded into a memory accessible to the processing unit.

The computer program product may have access to a memory storing a model for determining the timing of the interferential stimulation signal in relation to the input from the electrical signal sensor.

The apparatus of the first aspect may be used in a method for providing electrical stimulation of a heart, said method comprising: detecting, using an electrical signal sensor, conduction of an electrical signal in the heart; determining a timing of an interferential stimulation signal in relation to the conduction of the electrical signal in the heart; and outputting one or more control signals for controlling a first stimulation signal and a second stimulation signal in dependence of the determined timing, generating, by a stimulation generating unit, the first stimulation signal and the second stimulation signal, wherein the first stimulation signal has a first frequency and the second stimulation signal has a second frequency, outputting the first stimulation signal to a first pair of electrodes arranged in relation to a location in the heart and outputting the first stimulation signal to a second pair of electrodes arranged in relation to the location in the heart for forming an interferential stimulation signal in the location of the heart based on the first and second stimulation signals, wherein a difference between the first frequency and the second frequency defines a beat frequency of the interferential stimulation signal for controlling conduction of the electrical signal in the location in the heart.

Effects and features of this fourth aspect are largely analogous to those described above in connection with the first, second, and third aspects. Embodiments mentioned in relation to the fourth aspect are largely compatible with the first, second, and third aspects.

The method allows output of a stimulation signal that affects conduction of electrical signals in the heart. The stimulation signal may be controlled such that the stimulation signal is only output when needed and is controlled to be properly timed with electrical signals in the heart. The stimulation signal may interact with electrical signals being conducted in the heart so as to affect such electrical signals.

The method allows stimulation signals to be used that do not affect tissue such that no scars or any permanent damage to tissue are created. This implies that the method may directly affect the condition that needs to be treated (undesired conduction of electrical signals) without a need to physically prevent any conduction of electrical signals.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view illustrating interferential stimulation of a location in a body.
Fig. 2 is a schematic view of an apparatus according to an embodiment.
Fig. 2a is a schematic view of an interferential stimulation signal according to an embodiment.
Fig. 3 is a functional view of an apparatus according to an embodiment.
Fig. 4 is a flow chart of a method according to an embodiment.

### Detailed description

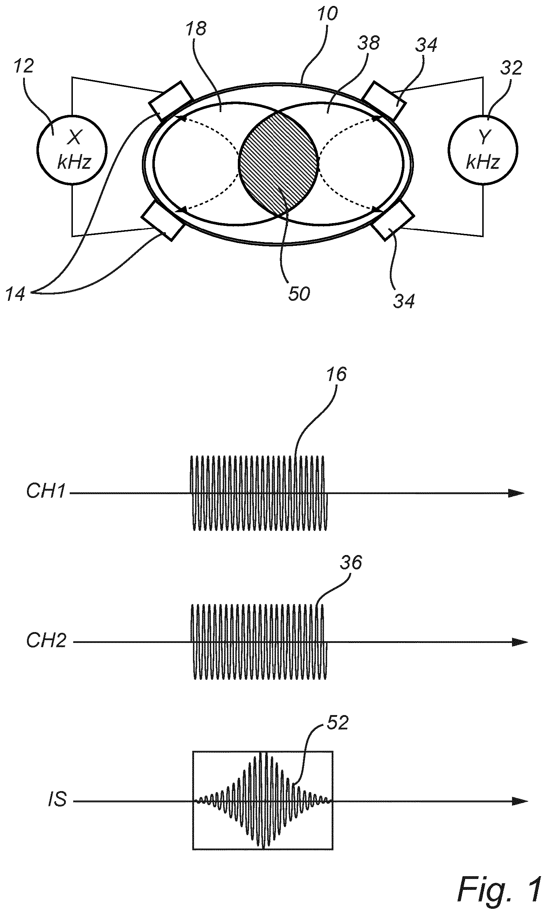

Referring now to Fig. 1, interferential stimulation of a location in a body will be generally described.

As illustrated in Fig. 1, a first signal generator 12 in form of a first current source is associated with a first pair of electrodes 14 and a second signal generator 32 in form of a second current source is associated with a second pair of electrodes 34. The first pair of electrodes 14 is mounted in a first relation to an outer surface of a body part 10, such as a heart, and the second pair of electrodes 34 is mounted in a second relation to the outer surface of the body part 10. It should be realized that the pairs of electrodes 14, 34, may be arranged in many different manners in relation to the location in the body in which stimulation is to be provided. It may be beneficial that the pairs of electrodes 14, 34 are arranged close to the location in which interferential stimulation is to be provided.

The first current source 12 generates a first stimulation signal 16 with a first frequency X kHz and the second current source 32 generates a second stimulation signal 36 with a second frequency Y kHz. As illustrated in Fig. 1, the first stimulation signal 16 provided to the first pair of electrodes 14 causes an electric field to be generated in a first portion 18 of the body part 10 and the second stimulation signal 36 provided to the second pair of electrodes 34 causes an electric field to be generated in a second portion 38 of the body part 10. The electric fields overlap in an overlapping portion 50, wherein the first signal 16 interferes with the second signal 36 to generate an interferential signal 52.

The interferential signal 52 has a frequency content including the first frequency and the second frequency. Further, the amplitude of the interferential signal 52 is modulated by a difference between the first frequency and the second frequency, X-Y, defining a beat frequency of the interferential signal 52.

The beat frequency may be much lower than the first and second frequencies. Further, the amplitude of the interferential signal 52 may be larger than individual amplitudes of the first signal 16 and the second signal 36, respectively, thanks to constructive interference of the signals.

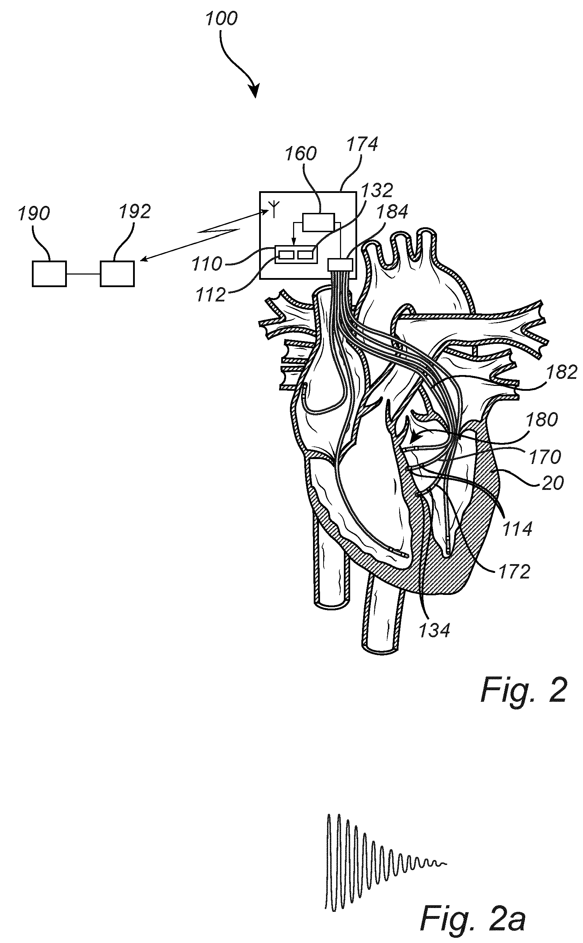

Referring now to Fig. 2, an apparatus 100 for controlling conduction of an electrical signal in a heart 20 will be described. The apparatus 100 is configured to provide interferential stimulation to the heart 20 as generally described above in relation to Fig. 1.

The apparatus 100 may be configured to provide interferential stimulation in a plurality of locations in the heart 20. This may allow stimulation of the plurality of locations to cooperate to aid the heart 20 in providing a desired conduction of electrical signals for controlling contraction and expansion of the heart 20.

For instance, the apparatus 100 may be configured to block conduction of electrical signals in one or more locations and may be configured to trigger conduction of electrical signals in one or more other locations. This may for instance be used for preventing or stopping arrhythmias and/or for strengthening proper conduction of electrical signals in the heart 20. The number of locations and the exact positions of these locations to be used for stimulation may be determined by a cardiac electrophysiologist and may be specific for each patient.

The apparatus 100 may provide control of conduction of electrical signals when the heart 20 needs help to provide a proper function. This implies that the apparatus 100 need not provide continuous stimulation but may rather be temporarily activated.

The apparatus 100 is configured to provide electrical signals for stimulation. Thus, when the apparatus 100 is not activated, the apparatus 100 may have no effect on the function of the heart 20. This implies that the apparatus 100 may have much less impact on the heart 20 compared to use of cardiac ablation for treating arrhythmias, wherein scars are created in heart tissue, which may locally degrade functionality of the heart muscle.

The apparatus 100 may comprise an electrical signal sensor 180, having one or more sensor units. The electrical signal sensor 180 may be configured to detect conduction of electrical signals in the heart 20. The apparatus 100 may comprise one or more sensor units close to each of the locations in which stimulation is to be provided. The apparatus 100 may also or alternatively comprise sensor unit(s) arranged in other locations in which no stimulation is to be provided.

Each sensor unit may be arranged in relation to heart tissue so as to be able to sense electrical activity in the heart. 20. The sensor unit may thus be configured for being mounted to a surface of the heart 20. The sensor unit(s) may be configured to detect electrical activity in the heart 20 which may be used as input for determining stimulation to be provided to the heart 20. The sensor unit(s) may thus detect desired electrical signals which should be strengthened by stimulation by the apparatus 100 for providing proper functionality of the heart 20. The sensor unit(s) may also or alternatively detect undesired electrical signals which should be blocked by stimulation by the apparatus 100 for providing proper functionality of the heart 20.

The apparatus 100 further comprises a control unit 160 which may be configured to determine the stimulation to be provided based on input received from the electrical signal sensor 180. The control unit 160 may be configured to predict an appropriate timing of stimulation of a location of the heart 20 such that the stimulation may block an undesired electrical signal when passing the location being stimulated and/or such that the stimulation may trigger or strengthen an electrical signal in the location in synchronization with the heart activity.

The control unit 160 may be configured to control stimulation in each location of the heart 20 that the apparatus 100 is able to stimulate. The input from a plurality of sensor units of the electrical signal sensor 180 may be used jointly for controlling the stimulation in each location. However, it should also be realized that the apparatus 100 may comprise a plurality of control units, each dedicated to controlling stimulation in a respective location of the heart 20. Each control unit may receive input from a respective sensor unit for controlling the stimulation in the respective location.

The control unit 160 may be configured to control stimulation by controlling output of stimulation signals from a stimulation generating unit 110. The apparatus 100 may comprise a plurality of stimulation generating units 110, each dedicated for generating stimulation signals for stimulation in a respective location of the heart 20. However, it should be realized that the apparatus 100 may alternatively comprise a single stimulation generating unit 110 which may be configured to generate the stimulation signals for stimulating in all locations of the heart 20.

As illustrated in Fig. 2 and discussed above, the apparatus 100 may be configured to provide stimulation in multiple locations of the heart 20. The apparatus 100 may thus comprise multiple sets of electrodes, each dedicated to providing stimulation in a respective location of the heart 20. However, for clarity and brevity, stimulation in one location of the heart 20 will now be described in detail. It should be realized that stimulation may be provided in a similar manner in each of the multiple locations of the heart 20 in which stimulation is enabled.

The apparatus 100 comprises the stimulation generating unit 110, which may comprise separate stimulation generating modules, each dedicated to generating stimulation signals to a respective pair of electrodes. Alternatively, the stimulation generating unit 110 may be a single unit configured to generate and output the stimulation signals to the electrodes.

In Fig. 2, the apparatus 100 is shown with the stimulation generating unit 110 comprising a first signal generator 112 and a second signal generator 132. The first signal generator 112 and the second signal generator 132 are separate from each other which may facilitate that the generation and output of stimulation signals from the signal generators 112, 132 may be individually provided without crosstalk between the signal generators 112, 132.

Each signal generator may comprise a current generator for generating a current signal. The current generator may comprise an oscillator, which is configured to generate an alternating signal and an amplifier, which is configured to receive the alternating signal from the oscillator and output an amplified signal. The signal generator may be controlled, e.g., for controlling a frequency of the alternating signal output by the oscillator or for controlling a gain of the amplifier. As realized by the person skilled in the art, the signal generator may be implemented in numerous other ways for generating an alternating signal.

The first signal generator 112 may comprise a first current generator, which may be connected to a first pair of electrodes 114. The first current generator may be configured to generate a first stimulation signal and to output the first stimulation signal to the first pair of electrodes 114. The first pair of electrodes 114 are arranged in a first relation to the location of the heart 20.

The second signal generator 132 may comprise a second current generator, which may be connected to a second pair of electrodes 134. The second current generator 132 may be configured to generate a second stimulation signal and to output the second stimulation signal to the second pair of electrodes 134. The second pair of electrodes 134 are arranged in a second relation to the location of the heart 20.

The first signal generator 112 and the second signal generator 132 are configured to generate and output the first stimulation signal comprising a first frequency and the second stimulation signal comprising a second frequency with a difference in the first and second frequencies such that the difference between the first frequency and the second frequency defines a beat frequency in interference within the location of the heart 20 of the first stimulation signal and the second stimulation signal.

The first signal generator 112 and the second signal generator 132 may be configured to generate the first stimulation signal and the second stimulation signal, respectively, having a first frequency and a second frequency, respectively, in a range of 500 Hz - 1 MHz, such as 5 kHz - 100 kHz.

The first signal generator 112 and the second signal generator 132 may further be configured to generate the first stimulation signal and the second stimulation signal with a difference in frequency in a range of 1 Hz - 10 kHz, such as 1 - 5 kHz. Thus, the beat frequency of the interferential stimulation signal may be in the range of 1 Hz - 10 kHz, such as 1 - 5 kHz.

The first frequency and the second frequency generated by the first signal generator 112 and the second signal generator 132 may thus be sufficiently high so as not to affect the heart 20, such as being higher than 500 Hz or higher than 50 kHz. The difference between the first frequency and the second frequency, i.e., the beat frequency, may be set so as to be adapted to provide proper stimulation of the heart 20.

The first stimulation signal and the second stimulation signal may be sinusoidal signals. This implies that no net charges are delivered to the living being by the first stimulation signal and the second stimulation signal, respectively. Hence, accumulation of net charges in the living being is avoided, which may otherwise negatively affect the living being.

The first pair of electrodes 114 and the second pair of electrodes 134, are configured to be arranged in relation to the location in the heart 20. The first pair of electrodes 114 and the second pair of electrodes 134 may be configured to be arranged on opposite sides of the location to be stimulated. For instance, the first pair of electrodes 114 and the second pair of electrodes 134 may be configured to be arranged on a surface of heart tissue facing into the heart tissue. The first pair of electrodes 114 and the second pair of electrodes 134 may be spaced apart along the surface of the heart tissue such that the location of the heart 20 to be stimulated is between the pairs of electrodes 114, 134 within the heart tissue.

Further, individual electrodes within a pair of electrodes 114, 134 may also be configured to be arranged with a distance in-between the electrodes such that the stimulation signal received by the electrodes will propagate through heart tissue between the electrodes.

Thus, the first pair of electrodes 114 may be associated with a first part of heart tissue at the location to be stimulated and the second pair of electrodes 134 may be associated with a second part of the heart tissue at the location. This implies that the portions of the cross-section of the heart tissue in which the first stimulation signal and the second stimulation signal, respectively, define electric fields are quite different so as to allow interferential stimulation deep within the heart tissue.

However, according to an alternative, the electrodes of the first pair of electrodes 114 may be configured to be alternatingly arranged with the electrodes of the second pair of electrodes 134 at the location of the heart 20 to be stimulated. Different arrangements of the first pair of electrodes 114 and the second pair of electrodes 134 in relation to the heart 20 may be used depending on a location within the heart 20 at which stimulation is desired.

As illustrated in Fig. 2, the apparatus 100 may comprise a plurality of carriers 170, 172. Each pair of electrodes 114, 134 may be arranged on a respective carrier 170, 172. The carriers 170, 172 may be flexible to allow the electrodes to be freely arranged in relation to the heart 20. The carriers 170, 172 may further be elongate to extend from a base unit 174, in which the stimulation generating unit 110 and the control unit 160 are arranged, to the location of the heart 20 to be stimulated.

Further, wires may be provided within the carriers 170, 172 so as to provide electrical connection between the pairs of electrodes 114, 134 and the stimulation generating unit 110.

The electrodes within a pair may for instance be arranged at different longitudinal positions within the elongate carrier 170, 172, providing a spacing between the electrodes.

However, it should be realized that the apparatus 100 may be configured in a different manner for placing the electrodes in relation to the heart 20. For instance, the electrodes may be arranged in a patch to be arranged at a surface of the heart 20, such that both the first pair of electrodes 114 and the second pair of electrodes 134 may be arranged in the same patch. The apparatus 100 may further comprise a tubing providing a connection between the patch and the base unit 174 for electrically connecting the electrodes to the stimulation generating unit 110.

As mentioned above, the apparatus 100 comprises a control unit 160. The control unit 160 may be configured to provide control signals to the stimulation generating unit 110 by providing control signals to the first signal generator 112 and the second signal generator 132 for controlling generation of the first stimulation signal and generation of the second stimulation signal. The control unit 160 may further be configured to synchronize the first signal generator 112 and the second signal generator 132 such that the first stimulation signal and the second stimulation signal are output simultaneously or almost simultaneously with a desired time relation to each other. The control unit 160 may thus ensure that a desired interferential signal is generated for providing desired interferential stimulation of the location of the heart 20.

The control unit 160 may be configured to provide control signals to the first signal generator 112 and to the second signal generator 132, respectively, for controlling the generation of the first stimulation signal and the generation of the second stimulation signal, respectively. The control signals to the first signal generator 112 and to the second signal generator 132 may modulate the first stimulation signal and the second stimulation signal, respectively, for controlling the interferential stimulation provided by the apparatus 100.

The control unit 160 may be configured to provide control signals for modulating the first frequency of the first stimulation signal and/or the second frequency of the second stimulation signal. This implies that the beat frequency may be changed.

The control unit 160 may be configured to provide control signals for modulating an amplitude of the first stimulation signal and/or an amplitude of the second stimulation signal. This implies that the portion within the location in the heart 20 having a maximum amplitude of the interferential signal may be moved. The portion having maximum amplitude of the interferential signal is located closer to a pair of electrodes outputting a weaker signal than to a pair of electrodes outputting a stronger signal.

The control unit 160 may be configured to provide control signals for modulating a time instant for start of the first stimulation signal and/or a time instant for start of the second stimulation signal. Thus, a phase relationship between the first stimulation signal and the second stimulation signal may be controlled for controlling a waveform of the interferential signal.

The control unit 160 is configured to receive input from the electrical signal sensor 180, which detects the conduction of electrical signals in the heart 20. The control unit 160 is further configured to control the timing of the interferential stimulation signal in relation to conduction of the electrical signal in the heart 20.

According to an embodiment, the control unit 160 may be implemented as a general-purpose processing unit, such as a central processing unit (CPU), which may execute the instructions of one or more computer programs in order to control the generation and timing of control signals. Hence, a computer program product may be provided, which provides computer-readable instructions for causing the control unit 160 to perform the processing. The computer program product may be provided as a signal carrying the computer program product for allowing the computer program product to be loaded into a memory accessible to the control unit 160. According to an embodiment, the computer program product may be provided as a non-transient computer program product stored on any tangible media.

The control unit 160 may further comprise a memory storing the application program and storing settings for controlling the generation and timing of control signals. The control unit 160 may be configured to receive new settings for altering the control by the control unit 160. The control unit 160 may also or alternatively comprise several versions of settings to allow selection of different settings depending on a desired stimulation. The control unit 160 may further comprise a clock or may be configured to receive a clock signal as input for timing of control signals.

According to an alternative, the control unit 160 may be implemented as firmware arranged e.g., in an embedded system, or as a specifically designed processing unit, such as an Application-Specific Integrated Circuit (ASIC) or a Field-Programmable Gate Array (FPGA), which may be configured to implement functionality for controlling the generation and timing of control signals.

The control unit 160 may be configured to control output of the first and second stimulation signals for controlling the interferential stimulation signal to block conduction of the electrical signal in the heart 20. The first and second pairs of electrodes 114, 134 may be arranged at a location through which an undesired pathway of conduction of electrical signals in the heart 20 passes. Thus, electrical signals may need to be blocked at the location in the heart 20. The control unit 160 may thus be configured to control output of the first and second stimulation signals such that the interferential stimulation signal interferes with the electrical signal being conducted through the location so as to attenuate the electrical signal and prevent the electrical signal from being passed through the location of the heart 20.

The control unit 160 may be configured to control output of the first and second stimulation signals for controlling the interferential stimulation signal to trigger conduction of the electrical signal in the heart 20. The first and second pairs of electrodes 114, 134 may be arranged at a location through which a desired pathway of conduction of electrical signals in the heart 20 passes. Thus, electrical signals may need to be conducted through the location in the heart 20. The control unit 160 may thus be configured to control output of the first and second stimulation signals such that the interferential stimulation signal triggers the electrical signal to be conducted through the location. The interferential stimulation signal may strengthen an electrical signal propagating in the desired pathway or may trigger generation of an electrical signal in the location.

It should be realized that different stimulation may be needed at different locations of the heart 20, such that the apparatus 100 may provide an interferential stimulation signal in a first location for blocking electrical signals through the first location and may provide an interferential stimulation signal in a second location for triggering electrical signals to be generated in the second location. Further, it should be realized that the control unit 160 may be configured to control forming of the interferential stimulation signal so as to provide different effects at different occasions of the interferential stimulation signal in a particular location. Thus, the interferential stimulation signal formed at the same location may at a first time instance block conduction of the electrical signal and at a second time instance trigger generation of the electrical signal.

The control unit 160 may be configured to determine the timing of the interferential stimulation signal in various manners in relation to input received from the electrical signal sensor 180.

For instance, the apparatus 100 may comprise an electrical signal sensor 180 that is arranged in close relation to the location of the heart 20 to be stimulated. Thus, the electrical signal sensor 180 may be arranged to detect electrical signals that are conducted at the location of the heart 20. The electrical signal sensor 180 may be configured to detect electrical signals propagating towards the location.

The electrical signal sensor 180 may comprise electrodes for detecting electrical signals. The electrodes may be configured to be arranged at a surface of the heart 20 in order to detect electrical signals being conducted through heart tissue. The electrodes may further be connected to read-out circuitry for reading out the signals detected by the electrodes. This may involve analog-to-digital conversion of the detected signals such that the electrical signal sensor 180 may be configured to provide digital input to the control unit 160.

The electrodes of the electrical signal sensor 180 may be arranged on a flexible carrier 182, similar to the carriers 170, 172, for facilitating arrangement of the electrodes in relation to a desired detection location. The flexible carrier 182 may be connected to the base unit 174, in which read-out circuitry 184 of the electrical signal sensor 180 may be provided. Further, wires may be provided within the carrier 182 so as to provide electrical connection between the electrodes and the read-out circuitry 184.

The electrodes of the electrical signal sensor 180 may be arranged in a common carrier with at least one of the first and second pairs of electrodes 114, 134. This may facilitate arranging the apparatus 100 in relation to the heart 20 as fewer carriers need to be fixed to the heart 20.

The control unit 160 may be configured to trigger output of the first and second stimulation signals based on the detection of an electrical signal by the electrical signal sensor 180. The control unit 160 may be configured to output the first and second stimulation signals such that the interferential stimulation signal is formed in the location of the heart 20 at the same time as the electrical signal is conducted through the location.

The control unit 160 may be configured to trigger output of the first and second stimulation signals with a fixed delay in relation to the detection of the electrical signal by the electrical signal sensor 180.

According to an alternative, the apparatus 100 may comprise two or more sensor units arranged at two different positions in close relation to the location of the heart 20 to be stimulated. The two sensor units may be arranged to detect electrical signals at two positions when propagating towards the location. Thus, a speed of conduction may be determined based on the detection in the two positions, which may be further used by the control unit 160 for determining timing of output of the first and second stimulation signals. With knowledge of distances between the two sensor units and the location to be stimulated, the control unit 160 may be able to determine an appropriate timing of the interferential stimulation signal.

Two or more sensor units may alternatively be used for detecting, at a first position, electrical signals propagating towards the location and detecting, at a second position, electrical signals when propagating from the location. Thus, the sensor unit detecting electrical signals propagating from the location may provide feedback for determining whether a desired effect of the stimulation has been provided. This may be used for adapting the control provided by the control unit 160 such that a time relation between detection of the electrical signal at the first position and output of the first and second stimulation signals may be adapted.

The control unit 160 may store a model for determining the timing of the interferential stimulation signal in relation to input from the electrical signal sensor 180.

For instance, the apparatus 100 may comprise multiple sensor units of the electrical signal sensor 180, which may be arranged at multiple locations in the heart 20 in order to detect electrical signals being conducted in multiple locations. The model stored by the control unit 160 may enable determining timing to be used for the interferential stimulation signal in the location(s) to be stimulated based on timing of detection of electrical signals in the multiple locations and the time relationship of the electrical signals in the multiple locations.

This may be particularly useful for controlling timing of interferential stimulation signals in multiple locations and ensuring that the interferential stimulation signals cooperate to provide a desired behavior of the heart 20.

The control unit 160 may provide a desired time relationship between interferential stimulation signals at different locations. The interferential stimulation signals at multiple locations may for instance cooperate to ensure conduction of electrical signals along a desired pathway by triggering generation of electrical signals in multiple locations with a desired time relationship and/or strengthening the electrical signal being conducted through the locations being stimulated.

The interferential stimulation signals at multiple locations may alternatively cooperate to ensure blocking of electrical signals along an undesired pathway by providing stimulation with a desired time relationship for attenuating the electrical signal to be blocked. In some instances, it may not be sufficient to provide stimulation at a single location for blocking an electrical signal. Rather, using two or more stimulation locations, the electrical signal may be completely blocked.

The stimulation generating unit 110 may be configured to generate a pulse of the first stimulation signal and a pulse of the second stimulation signal for forming an interferential stimulation signal having a duration of not more than two periods of the beat frequency. For instance, the stimulation generating unit 110 may be configured to generate the first stimulation signal and the second stimulation signal such that the interferential stimulation signal has a duration of a full period of the beat frequency, as illustrated in Fig. 1.

The interferential stimulation signal may thus include a rise in intensity followed by a fall in intensity. This may be suitable for stimulating the location of the heart. However, the location of the heart may be stimulated already by providing the interferential stimulation signal for a duration of only half a period of the beat frequency. For instance, the interferential stimulation signal may correspond to a rise in intensity. Such a stimulation signal is illustrated in Fig. 2a. However, the interferential stimulation signal may alternatively correspond to a fall in intensity during half a period of the beat frequency.

It should further be realized that the interferential stimulation signal may be even more accurately controlled using further stimulation signals. Thus, the apparatus 100 may comprise a third pair of electrodes, and possibly even further pairs of electrodes, configured to be arranged in relation to the location in the heart.

The stimulation generating unit 110 may then be configured to generate a third stimulation signal. For instance, the stimulation generating unit 110 may comprise a third signal generator, similar to the first and second signal generators, for generating the third stimulation signal. The third pair of electrodes is configured to receive the third stimulation signal. Thus, the interferential stimulation signal in the location of the heart may be based on the first, second, and third stimulation signals. The third stimulation signal may be used for modulating the interferential stimulation signal formed by interference of the first and second stimulation signals.

The third signal generator may be configured to generate the third stimulation signal comprising a third frequency which may be related to the beat frequency by an integer number.

According to an embodiment, the beat frequency may be two times larger than the third frequency. The beat frequency being two times larger than the third frequency may be particularly advantageous in forming a desired interferential signal in the location of the heart 20.

However, it should be realized that the third frequency may be related to the beat frequency by an integer number in other ways. For instance, the third frequency may be equal to the beat frequency, or the third frequency may be two, three or four times larger than the beat frequency.

Thanks to using a third stimulation signal with a third frequency related to the beat frequency as indicated above, the third stimulation signal may modulate the interference between the first and second stimulation signal so as to control a waveform of the interferential signal that may facilitate causing stimulation in the location of the heart 20.

Thus, according to embodiments, the third frequency of the third waveform may be in the range of 0.5 Hz - 20 kHz, such as 500 Hz - 10 kHz. The third stimulation signal may be a sinusoidal signal. This implies that no net charges are delivered to the living being by the third stimulation signal.

The third stimulation signal may be configured to modulate the interference between the first and the second stimulation signals such that an amplitude of the interferential stimulation signal is substantially increased. In addition, the interferential stimulation signal may be formed with a similar signal shape as a typical monophasic or biphasic current stimulation which is realized in existing implants.

The apparatus 100 as described above may be configured to be implanted in the body of the subject. For instance, the apparatus 100 may form part of a CRT device or an ICD device. In particular, the pairs of electrodes for providing stimulation to the location of the heart and the electrical signal sensor may be implanted to be arranged in close relation to the location of the heart 20 to be stimulated. Further, the stimulation generating unit 110 and the control unit 160 may advantageously be arranged implanted in the body. The stimulation generating unit 110 should be electrically connected to the electrodes and implanting the stimulation generating unit 110 facilitates connecting the stimulation generating unit 110 to the electrodes. Further, by also having the control unit 160 implanted, preferably in a common housing with the stimulation generating unit 110, control signals may be transferred quickly to the stimulation generating unit 110 to ensure proper timing of the interferential stimulation signal.

However, it should be realized that the apparatus 100 may comprise further components which need not necessarily be implanted in the body. For instance, the control unit 160 may be associated with a communication unit implanted in the body, for providing wireless communication with a component arranged externally to the body, such as being worn by the subject.

The apparatus 100 may comprise a sensor 190 for detecting information relating to functionality of the heart. The sensor 190 may for instance comprise a plurality of electrodes being configured to be arranged externally on the body of the subject for detecting an ECG.

The apparatus 100 may also comprise a processor 192, which may be arranged external to the subject. The processor 192 may be connected to the sensor 190 and may be configured to receive and analyze the information detected by the sensor 190.

Thus, the apparatus 100 may be configured to detect information externally to the body of the subject, which information may further be used for controlling stimulation. The processor 192 may be configured to analyze whether a desired effect of the stimulation is achieved. The processor 192 may thus provide input to the control unit 160 for adapting control by the control unit 160.

According to an embodiment, the processor 192 may be implemented as a general-purpose processing unit, such as a CPU, which may execute the instructions of one or more computer programs in order to perform analysis of the information detected by the sensor 190. According to an alternative, the processor 192 may be implemented as firmware arranged e.g., in an embedded system, or as a specifically designed processing unit, such as an ASIC or a FPGA, which may be configured to implement functionality for performing the analysis of the information detected by the sensor 190.

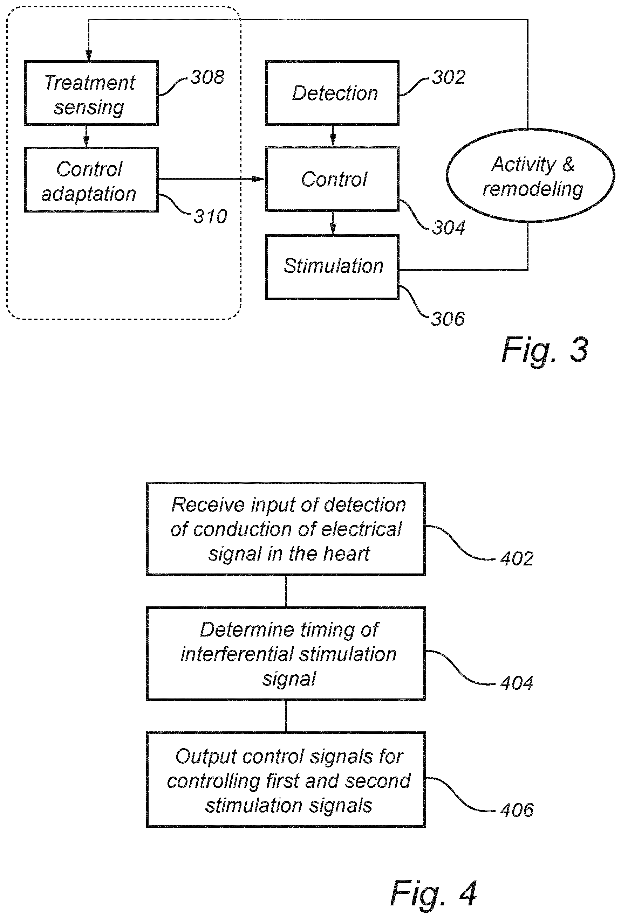

Referring now to Fig. 3, control of stimulation will be described.

The apparatus 100 may provide a detection function 302 for detecting electrical signals being conducted in the heart. For instance, electrical signals for atrial or ventricular activation may be detected.

The detection function 302 may provide input for controlling timing of stimulation by the apparatus 100. Thus, the apparatus 100 may further comprise a control function 304 which may be configured to determine a timing of stimulation. The timing of stimulation may provide pacing with regard to atrial or ventricular activation. The timing of stimulation may also or alternatively provide timing for blocking undesired electrical signals.

The apparatus 100 may further comprise a stimulation function 306 for providing the stimulation of the heart. The stimulation function 306 may be controlled by the timing provided by the control function 304 and may further be configured to output first and second stimulation signals for forming interferential stimulation at a location of the heart to be stimulated.

The stimulation provided by the apparatus 100 may thus affect activity of the heart and provide a remodeling of heart functionality.

The apparatus 100 may further comprise a treatment sensing function 308, which may be provided by a sensor externally to the body. The treatment sensing function 308 may be configured to detect a suitable biomarker for determining heart function. For instance, the treatment sensing function 308 may be configured to detect an ECG.

The treatment sensing function 308 may be configured to detect information to allow identifying whether the heart 20 functions properly. For instance, detection of an ECG may be used for identifying arrhythmias or a QRS duration of a heartbeat.

The apparatus 100 may further comprise a control adaptation function 310. The control adaptation function 310 may be configured to receive the information detected by the treatment sensing function 308. The control adaptation function 310 may use this information as feedback providing input of efficiency of stimulation and may analyze the feedback information for determining necessary adaptation of the stimulation. For instance, the control adaptation function 310 may determine any adaptations necessary for a time relation between the interferential stimulation signal and the electrical signals detected by the detection function 302. Thus, the control adaptation function 310 may provide input to the control function 304 for adapting timing of stimulation provided by the control function 304, e.g. for desired atrial or ventricular activation and pacing.

The control adaptation function 310 may also or alternatively determine necessary changes to amplitudes of the first and second stimulation signals for forming the interferential stimulation signal and/or phase differences between the first and second frequencies, which may change spatial position of the stimulation provided by the interferential stimulation signal. Thus, the control adaptation function 310 may also or alternatively provide such input to the control function 304 for adapting the stimulation.

Referring now to Fig. 4, a method for controlling conduction of an electrical signal in a heart is described.

The method comprises receiving 402 input from an electrical signal sensor configured to detect conduction of the electrical signal in the heart. The method further comprises determining 404 a timing of an interferential stimulation signal in relation to the conduction of the electrical signal in the heart. Thus, the method is able to determine timing of interferential stimulation signal such that a desired control of conduction of electrical signals in the heart may be provided based on a stimulation signal as opposed to, for instance, providing cardiac ablation wherein a permanent scar is provided to heart tissue for preventing undesired electrical signals to be conducted in the heart.

The method further comprises outputting 406 one or more control signals for controlling a first stimulation signal and a second stimulation signal in dependence of the determined timing. Thus, the method is able to control timing of generation of the first and second stimulation signals, which may further form the interferential stimulation signal.

The method may also include controlling amplitude, frequency and/or time relations between the first and second stimulation signals for providing further control of the stimulation signals to be generated. This may be used for controlling a spatial position of the interferential stimulation signal.

In the above the inventive concept has mainly been described with reference to a limited number of examples. However, as is readily appreciated by a person skilled in the art, other examples than the ones disclosed above are possible. The scope of the invention is defined by the appended claims.

## Claims

1. An apparatus (100) for controlling conduction of an electrical signal in a heart (20), said apparatus (100) comprising:
a stimulation generating unit (110) configured to generate a first stimulation signal and a second stimulation signal, wherein the first stimulation signal has a first frequency and the second stimulation signal has a second frequency;
a first pair of electrodes (114) configured to be arranged in relation to a location in the heart (20) in which conduction of the electrical signal is to be controlled, wherein the first pair of electrodes (114) is configured to receive the first stimulation signal;
a second pair of electrodes (134) configured to be arranged in relation to the location in the heart (20) in which conduction of the electrical signal is to be controlled, wherein the second pair of electrodes (134) is configured to receive the second stimulation signal, wherein the first and second pairs of electrodes (114, 134) are configured to form an interferential stimulation signal in the location of the heart (20) based on the first and second stimulation signals, wherein a difference between the first frequency and the second frequency defines a beat frequency of the interferential stimulation signal for controlling conduction of the electrical signal in the location in the heart (20);
an electrical signal sensor (180) configured to detect conduction of the electrical signal in the heart (20); and
a control unit (160) configured to receive input from the electrical signal sensor (180) and configured to control output of the first and the second stimulation signals for controlling a timing of the interferential stimulation signal in relation to the conduction of the electrical signal in the heart (20),
**characterized in that** the apparatus (100) is configured to be implanted in a body of a subject.

2. The apparatus according to claim 1, wherein the control unit (160) is configured to control output of the first and the second stimulation signals for controlling the interferential stimulation signal to block conduction of the electrical signal in the heart (20).

3. The apparatus according to claim 1, wherein the control unit (160) is configured to control output of the first and the second stimulation signals for controlling the interferential stimulation signal to trigger conduction of the electrical signal in the heart (20).

4. The apparatus according to any one of the preceding claims, wherein the stimulation generating unit (110) is configured to generate a pulse of the first stimulation signal and a pulse of the second stimulation signal for forming an interferential stimulation signal having a duration of not more than two periods of the beat frequency, such as a single period or half a period of the beat frequency.

5. The apparatus according to any one of the preceding claims, wherein the control unit is configured to store a model for determining the timing of the interferential stimulation signal in relation to the input from the electrical signal sensor or the control unit is configured to trigger output of the first and second stimulation signals based on detection of conduction of the electrical signal in the heart.

6. The apparatus according to any one of the preceding claims, wherein the electrical signal sensor (180) comprises multiple sensor units configured to detect electrical signals in multiple locations in the heart (20).

7. The apparatus according to any one of the preceding claims, wherein the electrical signal sensor (180) comprises electrodes configured to be arranged at the location of the heart (20) for detecting electrical signals conducted in the location of the heart (20).

8. The apparatus according to any one of the preceding claims, wherein the stimulation generating unit (110) comprises a first signal generator (112) associated with the first pair of electrodes (114) for generating the first stimulation signal and outputting the first stimulation signal to the first pair of electrodes (114) and wherein the stimulation generating unit (110) comprises a second signal generator (132) associated with the second pair of electrodes (134) for generating the second stimulation signal and outputting the second stimulation signal to the second pair of electrodes (134).

9. The apparatus according to any one of the preceding claims, wherein the control unit (160) is configured to control modulation of the interferential stimulation signal by modulating the first frequency and/or the second frequency, by modulating an amplitude of the first stimulation signal and/or an amplitude of the second stimulation signal, and/or by modulating a time instant for start of the first stimulation signal and/or a time instant for start of the second stimulation signal.

10. The apparatus according to any one of the preceding claims, further comprising a set of additional electrodes, wherein the set of additional electrodes comprises at least two pairs of electrodes configured to be arranged in relation to an additional location in the heart, wherein each pair of electrodes is configured to receive a respective stimulation signal for forming an interferential stimulation signal in the additional location of the heart based on the stimulation signals.

11. The apparatus according to any one of the preceding claims, wherein each pair of electrodes (114, 134) is arranged on a flexible, elongate carrier (170, 172), wherein the carrier (170, 172) comprises wires for connecting each electrode to the stimulation generating unit (110).

12. The apparatus according to any one of the preceding claims, further comprising a sensor (190) for detecting information relating to functionality of the heart (20) and a processor (192) configured to receive and analyze the information detected by the sensor (109) and to provide input to the control unit (160) for adapting control by the control unit (160).

13. A computer program product comprising computer-readable instructions such that when executed on a processing unit in the implantable apparatus according to any one of the preceding claims, the computer program product will cause the processing unit to perform a method for controlling conduction of an electrical signal in a heart, said method comprising:
receiving (402) input from an electrical signal sensor configured to detect conduction of the electrical signal in the heart;
determining (404) a timing of an interferential stimulation signal in relation to the conduction of the electrical signal in the heart; and
outputting (406) one or more control signals for controlling a first stimulation signal and a second stimulation signal in dependence of the determined timing.

## Patentansprüche

1. Vorrichtung (100) zum Steuern der Leitung eines elektrischen Signals in einem Herzen (20), wobei die Vorrichtung (100) umfasst:
eine Erregungserzeugungseinheit (110), die dazu konfiguriert ist, ein erstes Erregungssignal und ein zweites Erregungssignal zu erzeugen, wobei das erste Erregungssignal eine erste Frequenz aufweist und das zweite Erregungssignal eine zweite Frequenz aufweist;
ein erstes Elektrodenpaar (114), das dazu konfiguriert ist, mit Bezug auf eine Stelle in dem Herzen (20), in dem die Leitung des elektrischen Signals zu steuern ist, angeordnet zu sein, wobei das erste Elektrodenpaar (114) dazu konfiguriert ist, das erste Erregungssignal zu empfangen;
ein zweites Elektrodenpaar (134), das dazu konfiguriert ist, mit Bezug auf die Stelle in dem Herzen (20), in dem die Leitung des elektrischen Signals zu steuern ist, angeordnet zu sein, wobei das zweite Elektrodenpaar (134) dazu konfiguriert ist, das zweite Erregungssignal zu empfangen, wobei das erste und das zweite Elektrodenpaar (114, 134) dazu konfiguriert sind, ein Interferenzerregungssignal an der Stelle des Herzens (20) basierend auf dem ersten und dem zweiten Erregungssignal zu bilden, wobei eine Differenz zwischen der ersten Frequenz und der zweiten Frequenz eine Schlagfrequenz des Interferenzerregungssignals zum Steuern der Leitung des elektrischen Signals an der Stelle in dem Herzen (20) definiert;
einen elektrischen Signalsensor (180), der dazu konfiguriert ist, die Leitung des elektrischen Signals in dem Herzen (20) zu detektieren; und
eine Steuereinheit (160), die dazu konfiguriert ist, eine Eingabe von dem elektrischen Signalsensor (180) zu empfangen, und dazu konfiguriert ist, die Ausgabe des ersten und des zweiten Erregungssignals zu steuern, um eine Zeiteinstellung des Interferenzerregungssignals mit Bezug auf die Leitung des elektrischen Signals in dem Herzen (20) zu steuern,
**dadurch gekennzeichnet, dass** die Vorrichtung (100) dazu konfiguriert ist, in einem Körper einer Person implantiert zu sein.

2. Vorrichtung nach Anspruch 1, wobei die Steuereinheit (160) dazu konfiguriert ist, die Ausgabe des ersten und des zweiten Erregungssignals zu steuern, um das Interferenzerregungssignal zu steuern, um die Leitung des elektrischen Signals in dem Herzen (20) zu blockieren.

3. Vorrichtung nach Anspruch 1, wobei die Steuereinheit (160) dazu konfiguriert ist, die Ausgabe des ersten und des zweiten Erregungssignals zu steuern, um das Interferenzerregungssignal zu steuern, um die Leitung des elektrischen Signals in dem Herzen (20) auszulösen.

4. Vorrichtung nach einem der vorhergehenden Ansprüche, wobei die Erregungserzeugungseinheit (110) dazu konfiguriert ist, einen Impuls des ersten Erregungssignals und einen Impuls des zweiten Erregungssignals zu erzeugen, um ein Interferenzerregungssignal zu bilden, das eine Dauer von nicht mehr als zwei Perioden der Schlagfrequenz aufweist, wie etwa eine einzige Periode oder eine halbe Periode der Schlagfrequenz.

5. Vorrichtung nach einem der vorhergehenden Ansprüche, wobei die Steuereinheit dazu konfiguriert ist, ein Modell zum Bestimmen der Zeiteinstellung des Interferenzerregungssignals mit Bezug auf die Eingabe von dem elektrischen Signalsensor zu speichern, oder die Steuereinheit dazu konfiguriert ist, die Ausgabe des ersten und des zweiten Erregungssignals basierend auf der Detektion der Leitung des elektrischen Signals in dem Herzen auszulösen.

6. Vorrichtung nach einem der vorhergehenden Ansprüche, wobei der elektrische Signalsensor (180) mehrere Sensoreinheiten umfasst, die dazu konfiguriert sind, elektrische Signals an mehreren Stellen in dem Herzen (20) zu detektieren.

7. Vorrichtung nach einem der vorhergehenden Ansprüche, wobei der elektrische Signalsensor (180) Elektroden umfasst, die dazu konfiguriert sind, an der Stelle des Herzens (20) angeordnet zu sein, um elektrische Signale zu detektieren, die an der Stelle des Herzens (20) geleitet werden.

8. Vorrichtung nach einem der vorhergehenden Ansprüche, wobei die Erregungserzeugungseinheit (110) einen ersten Signalgeber (112) umfasst, der dem ersten Elektrodenpaar (114) zugeordnet ist, um das erste Erregungssignal zu erzeugen und das erste Erregungssignal an das erste Elektrodenpaar (114) auszugeben, und wobei die Erregungserzeugungseinheit (110) einen zweiten Signalgeber (132) umfasst, der dem zweiten Elektrodenpaar (134) zugeordnet ist, um das zweite Erregungssignal zu erzeugen und das zweite Erregungssignal an das zweite Elektrodenpaar (134) auszugeben.

9. Vorrichtung nach einem der vorhergehenden Ansprüche, wobei die Steuereinheit (160) dazu konfiguriert ist, die Modulation des Interferenzerregungssignals durch Modulieren der ersten Frequenz und/oder der zweiten Frequenz, durch Modulieren einer Amplitude des ersten Erregungssignals und/oder einer Amplitude des zweiten Erregungssignals, und/oder durch Modulieren eines Zeitpunkts zum Starten des ersten Erregungssignals und/oder eines Zeitpunkts zum Starten des zweiten Erregungssignals zu steuern.

10. Vorrichtung nach einem der vorhergehenden Ansprüche, ferner umfassend eine Menge von zusätzlichen Elektroden, wobei die Menge von zusätzlichen Elektroden mindestens zwei Elektrodenpaare umfasst, die dazu konfiguriert sind, mit Bezug auf eine zusätzliche Stelle in dem Herzen angeordnet zu sein, wobei jedes Elektrodenpaar dazu konfiguriert ist, ein jeweiliges Erregungssignal zu empfangen, um ein Interferenzerregungssignal an der zusätzlichen Stelle des Herzens basierend auf den Erregungssignalen zu bilden.

11. Vorrichtung nach einem der vorhergehenden Ansprüche, wobei jedes Elektrodenpaar (114, 134) auf einem biegsamen, länglichen Träger (170, 172) angeordnet ist, wobei der Träger (170, 172) Drähte umfasst, um jede Elektrode mit der Erregungserzeugungseinheit (110) zu verbinden.

12. Vorrichtung nach einem der vorhergehenden Ansprüche, ferner umfassend einen Sensor (190) zum Detektieren von Informationen bezüglich der Funktionsfähigkeit des Herzens (20) und einen Prozessor (192), der dazu konfiguriert ist, die von dem Sensor (109) detektierten Informationen zu empfangen und zu analysieren, und um eine Eingabe für die Steuereinheit (160) bereitzustellen, um die Steuerung durch die Steuereinheit (160) anzupassen.

13. Computerprogrammprodukt, umfassend computerlesbare Anweisungen, so dass, wenn es auf einer Verarbeitungseinheit in der implantierbaren Vorrichtung nach einem der vorhergehenden Ansprüche ausgeführt wird, das Computerprogrammprodukt bewirkt, dass die Verarbeitungseinheit ein Verfahren zum Steuern der Leitung eines elektrischen Signals in einem Herzen ausführt, wobei das Verfahren umfasst:
Empfangen (402) einer Eingabe von einem elektrischen Signalsensor, der dazu konfiguriert ist, die Leitung des elektrischen Signals in dem Herzen zu detektieren;
Bestimmen (404) einer Zeiteinstellung eines Interferenzerregungssignals in Bezug auf die Leitung des elektrischen Signals in dem Herzen; und
Ausgeben (406) eines oder mehrerer Steuersignale zum Steuern eines ersten Erregungssignals und eines zweiten Erregungssignals in Abhängigkeit von der bestimmten Zeiteinstellung.

## Revendications

1. Appareil (100) pour contrôler la conduction d'un signal électrique dans un cœur (20), ledit appareil (100) comprenant :
une unité de génération de stimulation (110) configurée pour générer un premier signal de stimulation et un deuxième signal de stimulation, dans lequel le premier signal de stimulation a une première fréquence et le deuxième signal de stimulation a une deuxième fréquence ;
une première paire d'électrodes (114) configurée pour être disposée par rapport à un emplacement dans le cœur (20) où la conduction du signal électrique doit être contrôlée, dans lequel la première paire d'électrodes (114) est configurée pour recevoir le premier signal de stimulation ;
une deuxième paire d'électrodes (134) configurée pour être disposée par rapport à l'emplacement dans le cœur (20) où la conduction du signal électrique doit être contrôlée, dans lequel la deuxième paire d'électrodes (134) est configurée pour recevoir le deuxième signal de stimulation, dans lequel la première et la deuxième paires d'électrodes (114, 134) sont configurées pour former un signal de stimulation interférentielle à l'emplacement du cœur (20) sur la base du premier et du deuxième signaux de stimulation, dans lequel une différence entre la première fréquence et la deuxième fréquence définit une fréquence de battement du signal de stimulation interférentielle pour contrôler la conduction du signal électrique à l'emplacement dans le cœur (20) ;
un capteur de signal électrique (180) configuré pour détecter la conduction du signal électrique dans le cœur (20) ; et
une unité de commande (160) configurée pour recevoir l'entrée du capteur de signal électrique (180) et configurée pour commander la sortie du premier et du deuxième signaux de stimulation afin de contrôler une synchronisation du signal de stimulation interférentielle par rapport à la conduction du signal électrique dans le cœur (20),
**caractérisé en ce que** l'appareil (100) est configuré pour être implanté dans un corps d'un sujet.

2. Appareil selon la revendication 1, dans lequel l'unité de commande (160) est configurée pour commander la sortie des premier et deuxième signaux de stimulation afin de contrôler le signal de stimulation interférentielle pour bloquer la conduction du signal électrique dans le cœur (20).

3. Appareil selon la revendication 1, dans lequel l'unité de commande (160) est configurée pour commander la sortie du premier et du deuxième signaux de stimulation afin de contrôler le signal de stimulation interférentielle pour déclencher la conduction du signal électrique dans le cœur (20).

4. Appareil selon une quelconque des revendications précédentes, dans lequel l'unité de génération de stimulation (110) est configurée pour générer une impulsion du premier signal de stimulation et une impulsion du deuxième signal de stimulation afin de former un signal de stimulation interférentielle d'une durée n'excédant pas deux périodes de la fréquence de battement, telle qu'une période entière ou une demi-période de la fréquence de battement.

5. Appareil selon une quelconque des revendications précédentes, dans lequel l'unité de commande est configurée pour mémoriser un modèle pour déterminer la synchronisation du signal de stimulation interférentielle par rapport à l'entrée du capteur de signal électrique ou l'unité de commande est configurée pour déclencher la sortie des premier et deuxième signaux de stimulation sur la base de la détection de la conduction du signal électrique dans le cœur.

6. Appareil selon une quelconque des revendications précédentes, dans lequel le capteur de signal électrique (180) comprend plusieurs unités de capteur configurées pour détecter les signaux électriques en plusieurs emplacements du cœur (20).

7. Appareil selon une quelconque des revendications précédentes, dans lequel le capteur de signal électrique (180) comprend des électrodes configurées pour être disposées à l'emplacement du cœur (20) afin de détecter les signaux électriques conduits à l'emplacement du cœur.

8. Appareil selon une quelconque des revendications précédentes, dans lequel l'unité de génération de stimulation (110) comprend un premier générateur de signal (112) associé à la première paire d'électrodes (114) pour générer le premier signal de stimulation et sortir le premier signal de stimulation vers la première paire d'électrodes (114) et dans lequel l'unité de génération de stimulation (110) comprend un deuxième générateur de signal (132) associé à la deuxième paire d'électrodes (134) pour générer le deuxième signal de stimulation et sortir le deuxième signal de stimulation vers la deuxième paire d'électrodes (134).

9. Appareil selon une quelconque des revendications précédentes, dans lequel l'unité de commande (160) est configurée pour commander la modulation du signal de stimulation interférentielle en modulant la première fréquence et/ou la deuxième fréquence, en modulant une amplitude du premier signal de stimulation et/ou une amplitude du deuxième signal de stimulation et/ou en modulant un instant de début du premier signal de stimulation et/ou un instant de début du deuxième signal de stimulation.

10. Appareil selon une quelconque des revendications précédentes, comprenant en outre un ensemble d'électrodes supplémentaires, dans lequel l'ensemble d'électrodes supplémentaires comprend au moins deux paires d'électrodes configurées pour être disposées par rapport à un emplacement supplémentaire dans le cœur, dans lequel chaque paire d'électrodes est configurée pour recevoir un signal de stimulation respectif afin de former un signal de stimulation interférentielle à l'emplacement supplémentaire du cœur, sur la base des signaux de stimulation.

11. Appareil selon une quelconque des revendications précédentes, dans lequel chaque paire d'électrodes (114, 134) est disposée sur un support flexible et allongé (170, 172), dans lequel le support (170, 172) comprend des fils pour connecter chaque électrode à l'unité de génération de stimulation (110).

12. Appareil selon une quelconque des revendications précédentes, comprenant en outre un capteur (190) pour détecter les informations relatives au fonctionnement du cœur (20) et un processeur (192) configuré pour recevoir et analyser les informations détectées par le capteur (109) et pour fournir une entrée à l'unité de commande (160) afin que l'unité de commande (160) adapte la commande.

13. Produit de programme informatique comprenant des instructions lisibles par ordinateur, de sorte que, lorsqu'il est exécuté sur une unité de traitement de l'appareil implantable selon une quelconque des revendications précédentes, le produit de programme informatique amène l'unité de traitement à exécuter un procédé de contrôle de la conduction d'un signal électrique dans un cœur, ledit procédé comprenant :
la réception (402) d'une entrée provenant d'un capteur de signal électrique configuré pour détecter la conduction du signal électrique dans le cœur ;
la détermination (404) d'une synchronisation d'un signal de stimulation interférentielle par rapport à la conduction du signal électrique dans le cœur ; et
la sortie (406) d'un ou plusieurs signaux de commande pour commander un premier signal de stimulation et un deuxième signal de stimulation en fonction de la synchronisation déterminée.
